# EUROPEAN PATENT APPLICATION

(11) **EP 3 424 447 A1**
(43) Date of publication of application: **09.01.2019**
(21) Application number: 17180346.3
(22) Date of filing: 07.07.2017
(51) Int. Cl.: A61B 17/34, A61B 90/00, A61B 17/00

(54) **MEDICAL NEEDLE DEVICE**

(71) Applicant: Plogmark, Oscar, 126 48 Hägersten (SE); Jonsson, Stefan, 192 70 Sollentuna (SE); Möller, Björn, 116 20 Stockholm (SE); Sundin, Victor, 131 31 Nacka (SE)
(72) Inventor: PLOGMARK, Oscar, 126 48 Hägersten (SE); MÖLLER, Björn, 116 20 Stockholm (SE)
(74) Representative: Bjerkéns Patentbyrå KB (Stockholm)

(57) **Abstract**

A medical needle device (2) comprising
- a needle holder (4) structured to releasably hold a medical needle (6);
- a needle movement generator (8) configured to apply movement to the needle (6) via said needle holder (4), wherein said movement is one or many of a rotating or oscillating movement about a longitudinal axis A of the needle (6), and an oscillating movement along said longitudinal axis A, and
- a control unit (10) configured to determine and to control said movement of the needle (6) by applying a movement control signal (12), including control instructions, to the needle movement generator (8) to activate or deactivate said movement.

The medical needle device (2) further comprises at least one parameter sensing member (14) configured to sense at least one parameter related to the needle (6), to generate at least one parameter signal (16) including parameter values representing at least one sensed parameter and to apply said parameter signal (16) to said control unit (10). The control unit (10) is configured to receive said parameter signal (16) and to apply a set of control rules to said parameter values to determine said control instructions.

## Description

### Technical field

The present disclosure relates to a medical needle device, which generally is applicable for spinal cord insertion procedures. The medical needle device is in particular useful in lumbar puncture procedures where the needle is inserted between lumbar vertebras.

### Background

Lumbar puncture is considered to be a routine technical procedure, and is used as a diagnostic tool, where Cerebrospinal Fluid (CSF) is tapped and analyzed for e.g. meningitis, encephalitis and subarachnoid Hemorrhage. Lumbar puncture is also used as a procedure for injecting epidural and spinal anesthesia. The procedure is performed using multiple different needles, where all needles have a central stylet to prevent debris or tissue from entering the needle. The stylet also reduces the risk of introducing epidermoid tissue into the spinal canal. The physician identifies the correct spot to perform the procedure, which is made in the lower lumbar region where the risk of neurological injury is minimized. The lumbar puncture process is conducted manually by a physician, who identifies a spot where a needle can be inserted in between the lumbar vertebras.

The procedure is prone to failure as the needle can be deflected by hard tissue such as calcified ligaments or bone. This results in frequent repetition of insertion procedure which naturally is negative for the patient, but also extends the duration of the procedure, and adds on cost to the procedure.

An intra-osseous needle drill is disclosed in US-5554154 which comprises a drill for a medical needle having a fitting end and a tip suitable for intra-osseous use and includes a rotatable chuck extending outwardly from a hand-held housing. The chuck includes means for releasably receiving the needle at its fitting end. Rotation of the chuck and needle may be powered electrically by a small motor in a housing. It is also mentioned that the chuck may be applied to any type of needles suitable for drilling action, e.g. a lumbar puncturing needle.

The object of the present invention is to achieve an improved medical needle device capable of performing an insertion procedure applied in diagnostic and therapeutic procedures involving insertion of a needle between vertebras. A particular object is to thereby achieve an improved lumbar puncture procedure.

### Summary

The above-mentioned object is achieved by the present invention according to the independent claim.

Preferred embodiments are set forth in the dependent claims.

The medical needle device according to the present invention was developed for the purpose of increasing the success rate of a lumbar puncture process by the use of applying a movement to the needle, e.g. a rotational movement, i.e. drilling, in combination with a feedback function, i.e. controlling movement of the needle in dependence of a sensed parameter related to the needle. Tests have been successfully conducted using standard Quincke needles that indicated that it would be possible to drill through calcified ligaments or even bone, which would otherwise deflect a needle. By sensing the applied torque and/or axial force, the mechanical stress of the needle was possible to determine. The rotation speed may together with the axial force be used for estimating the friction power loss to prevent thermal necrosis, i.e. heat related tissue damage. The use of a force sensor also enables automatic activation and deactivation of the rotation function, where it is only intended to be activated when hard tissue has been struck - tissue that cannot be pierced without the use of drilling. When the hard tissue has been pierced, the drill function is deactivated to prevent unnecessary damage to soft tissue. Thus, one essential object achieved by the medical needle device according to the present invention is to prevent the needle from breaking.

### Brief description of the drawings

Figure 1 is a schematic illustration of a medical needle device according to the present invention.
Figure 2 is a block diagram of a control unit according to the present invention.
Figure 3 is a schematic illustration of an embodiment of the present invention.
Figure 4 is a diagram illustrating two embodiments using a force sensor.
Figure 5 is a diagram illustrating an embodiment using a torque sensor.

### Detailed description

The medical needle device will now be described in detail with references to the appended figures. Throughout the figures the same, or similar, items have the same reference signs. Moreover, the items and the figures are not necessarily to scale, emphasis instead being placed upon illustrating the principles of the invention.

Before describing the medical needle device in detail a brief description of the anatomy and the medical procedure is included, where the medical needle device is particularly useful.

The spinal cord extends to the lower level of vertebras L1, and L2 interspace in 31% of adult patients. Therefore, lumbar puncture is generally performed in the L3-S1 interspaces, where there is little or no risk of neurological damage. In order to avoid overlying bone, the needle has to be inserted at a slight angle relative to the sagittal plane. In order to widen the gap between the vertebras, the spine is often flexed. The needle pierces in the following order in the midline approach skin, subcutaneous tissue, supraspinous ligament, ligamentum flavum, epidural space, dura, arachnoid and finally the subarachnoid space.
The supraspinous and interspinous ligaments as well as ligamentum flavum provide resistance, and a slight pop is often felt as the ligaments are penetrated. In elderly patients the ligaments are often calcified and therefore provide significant resistance and can in some cases deflect the needle. By placing the needle slightly laterally, at an angle of 10-20° the needle can be inserted using the so-called paramedian approach. By doing so, the supraspinous ligament and interspinous ligaments can be avoided. However, as the needle passes through the para-spinal muscles it hits the ligamentum flavum directly, which is a strong yellow elastic ligament. The ligamentum flavum can be up to 1 cm thick in the lumbar region, and it can also be calcified. Therefore, no matter where a needle is being inserted there is a risk for an unsuccessful attempt due to deflection.

The two basic needle types that exist to perform lumbar puncture are either cutting (Quincke) or noncutting (Pencil point) needles.

The typical characteristic of the Quincke needle is the 14° bevel as well as the 60° point angle, with the sharp cutting edges.
The pencil point needle has a 20° point angle and an opening on the side.

When performing puncture of dura mater one requirement is to minimize risk for complications which favors a dural hole having as small diameter as possible. This must be balanced by achieving a dural hole diameter for providing sufficient CSF flow rate to allow for pressure measurement as well as CSF collection.

A thinner needle is more likely to be deflected due to weaker mechanical properties. A 20- to 22-gauge, i.e. 0.6 mm to 0.8 mm, pencil point needle seems to be the best overall choice when all of these characteristics have been considered.

A stylet is normally provided, and is a wire running through the entire length of the needle, rendering it stiffer and preventing debris from entering the needle opening.

Bone is a calcified connective tissue, and able to heal itself by producing new bone-forming cells and blood vessels where a fracture has occurred. Drilling of bone is commonly used in orthopedic surgery to produce holes for screw insertion. A common problem is avoiding the threshold for thermal osteonecrosis i.e. bone death, which can delay the healing process negatively. In worst case, the bone could be broken down with decreased structural properties as a result. Controlling the heat the bone is subjected to is therefore critical for the healing process.
However, a threshold seems to be 47°C, above which thermal necrosis of the bone occurs. Thus, keeping the temperature well below this level is therefore argued necessary to shorten the recovery time of the patient.

The medical needle device according to the present invention will now be described in detail.

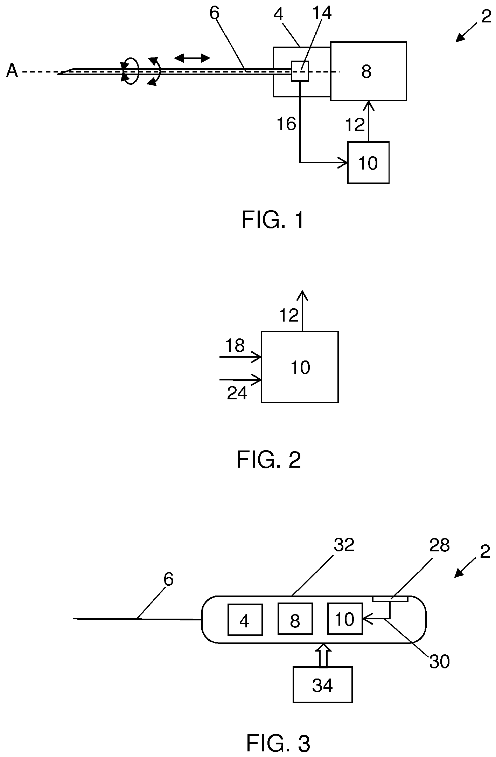

With references to figure 1, a medical needle device 2 is provided that comprises a needle holder 4 structured to releasably hold a medical needle 6. The medical needle may be a needle specifically adapted for a lumbar puncturing procedure, or more generally, a medical needle adapted to be applied during any spinal cord insertion procedure. The needle may e.g. be of any of the types discussed above, e.g. a cutting (Quincke) or noncutting (Pencil point) needle.

The medical needle device further comprises a needle movement generator 8 configured to apply movement to the needle 6 via the needle holder 4. The movement is one or many of a rotating or oscillating movement about a longitudinal axis A of the needle 6, and an oscillating movement along the longitudinal axis A. These movements are schematically illustrated by double-arrows in the figure. The needle movement generator is preferably an electrical motor, but the movements may also be generated by pressurized air or by a hydraulic member.

Furthermore, the medical needle device comprises a control unit 10 configured to determine and to control the movement of the needle 6 by applying a movement control signal 12, including control instructions, to the needle movement generator 8 to activate or deactivate the movement. The control unit 10 is provided with processing capabilities, and comprises e.g. a processor, and memory means.

The medical needle device 2 further comprises at least one parameter sensing member 14 configured to sense at least one parameter related to the needle 6, to generate at least one parameter signal 16 including parameter values representing the at least one sensed parameter and to apply the parameter signal 16 to the control unit 10. The at least one parameter sensing member is configured to sense the at least one parameter both when no movement is generated by the needle movement generator, and when movement is generated by the needle movement generator.
The control unit 10 is configured to receive the parameter signal 16 and to apply a set of control rules to the parameter values to determine the control instructions.

Preferably, the set of control rules includes at least one rule comprising to compare the parameter values to predetermined parameter thresholds, and that the control unit 10 is configured to control needle movement in dependence of the result of the comparison.

According to one embodiment the parameter sensing member 14 is a force sensing member configured to sense a force to the needle 6 along the longitudinal axis A and in a proximal direction of the needle.
The parameter sensing member 14 is configured to generate a force sensor signal 18 including force values F representing sensed forces and to apply the force sensor signal to the control unit 10. The control unit is configured to receive the force sensor signal 18 and to compare the force values to predetermined force thresholds, wherein the control unit is configured to control needle movement in dependence of the result of the comparison.

The cortical bone (white, denser bone) is tougher to penetrate which is indicated by starting and ending spikes in the measured force values. The spongy inside of the bone is not as difficult to drill through, but still needs the movement function, e.g. the drilling function, activated for the needle to be able to penetrate. Once the needle is through the bone the axial force drops, which indicates that an axial force threshold level can be used for activation and deactivation of the drilling process.

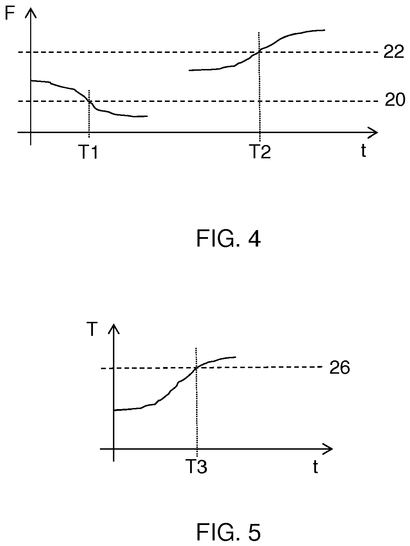

According to one embodiment illustrated by the diagram in figure 4 and in particular the left curve, if movement of the needle 6 is activated and a force value F is below a predetermined deactivation threshold 20 the control unit 10 is configured to generate the movement control signal 12 to the needle movement generator 8 to deactivate the movement. This situation may occur if the needle e.g. rotates and thus performs a drilling action. If the needle reaches softer tissue and therefore no drilling is required the drilling is stopped. This is illustrated at the point of time T1.
According to another embodiment also illustrated by the diagram in figure 4 and in particular the right curve, if movement of the needle 6 is deactivated and a force value is above a predetermined activation threshold 22 the control unit 10 is configured to generate the movement control signal 12 to the needle movement generator 8 to activate the movement. This situation may occur when the needle reaches harder tissue, e.g. a bone, and therefore a movement, e.g. a rotational movement, of the needle is required in order to penetrate the bone. This is illustrated at the point of time T2.

According to one example, the force sensing member is of strain gauge type. Also piezoelectric and piezoresistive force sensors may be applied. However, piezoresistive force sensors indicates a change of electrical resistance, and are also measured in a Wheatstone configuration, similar to the strain gauge. Moreover, piezoresistive force sensors can be made much smaller than strain gauges, which would be advantageous. Therefore, a piezoresistive force sensor, with a force range of 0-15N is one particularly useful sensor.

According to an embodiment the movement is a rotation about the longitudinal axis A. The rotational speed is dependent of the type of the tissue. Preferably it is within the range of 200 - 700 revolutions per minute (rpm), and more preferred within the range of 400 - 600 rpm. The speed may be variable, and may then be adapted to the hardness of the tissue.

According to another embodiment the movement is an oscillation about the longitudinal axis A, and that the oscillation is less than one revolution, and preferably less than half of a revolution. Preferably the oscillation speed it is within the range of 200 - 700 oscillation cycles per minute, and more preferred within the range of 400 - 600. Also this speed may be variable.

According to still another embodiment the movement instead is an oscillation along the longitudinal axis A having a predetermined stroke length. Preferably, the stroke length is within the range of 0.1 - 2.0 mm, and the number of longitudinal oscillations per minute is preferably within the range of 200 - 700 oscillations per minute, and more preferred within the range of 400 - 600. The number may be variable.

The longitudinal movement may be combined with the rotational or oscillating rotational movement.

According to another embodiment the parameter sensing member 14 is a torque sensing member configured to sense torque of the medical needle about the longitudinal axis A. The torque sensing member is configured to generate a torque sensor signal 24 including torque values T representing sensed torque and to apply the torque sensor signal 24 to the control unit 10. The control unit 10 is then configured to control needle movement in dependence of sensed torque values. The control unit 10 is configured to receive the torque sensor signal 24 and to compare the torque values to predetermined torque threshold values, and if movement of the needle is activated and a torque value is above a predetermined deactivation torque threshold 26 the control unit is configured to generate the movement control signal 12 to the needle movement generator 8 to deactivate the movement. This case is illustrated in the diagram shown in figure 6, where exemplary torque values are shown over time. In the point of time T3 the torque value exceeds the deactivation torque threshold 26 and the movement is deactivated in order to prevent the needle from breaking, or to keep tissue temperature increase below a specified threshold. This will be further discussed below.
In one variation the torque is determined by measuring a parameter representing the power consumption of the movement generator, e.g. the current. A current sensor is then used for sensing the current consumed by the electrical motor, and the sensed current is used in a conversion process in the control unit where it is converted into applied torque to the needle.

The at least one parameter sensing member 14 may be configured to sense other parameters related to the needle apart from force and torque, and combinations thereof, which have been mentioned above. For example, various parameters related to the rotation of the needle, e.g. acceleration, velocity, and/or rotational speed. These parameters may then be further processed in order to calculate power and energy.

When applying movement to the needle the temperature increase of tissue caused by the movement must be kept as low as possible. A maximum acceptable temperature is approximately 47 °C. Therefore, the control unit 10 is configured to determine the movement control signal 12 in dependence of the present movement, preferably dependent of a present rotational speed of the needle, the force values and/or the torque values, such that temperature increase in tissue caused by needle movement is below a predefined temperature increase threshold. This predefined temperature increase threshold is 4-8 °C.

With reference to the schematic illustration of figure 3, the medical needle device 2 further comprises a hand-held housing 32 structured to enclose the control unit 10 and the movement generator 8. The housing 32 has preferably a shape and a size that is adapted for one-hand use. It is e.g. made from a suitable plastic material. The medical needle device further comprises a power source 34, e.g. a rechargeable battery, or a connection to mains, configured to supply operating energy to the various parts of the device, e.g. the control unit and the movement generator. The power source 34 may be included in the housing 32, but may also be a separate unit connected to the housing via an electrical cable.
An activation member 28 is preferably provided, e.g. a button or similar, to receive an activation command from an operator, and wherein the control unit 10 is configured to receive an activation signal 30 from the activation member 28 and to activate movement in dependence thereto.

As discussed above, the medical needle device described herein is generally configured to be applied during a spinal cord insertion procedure. Particularly, it is configured to be applied during a lumbar puncturing procedure.

In exemplary tests, 0.7mm Quincke needles were used, both in their original shape and as a basis for alternative shapes, for drilling through the vertebrae body of domestic swine. It was concluded that it is possible to drill through the vertebrae body using the Quincke needle. The properties of a twist drill when drilling through the vertebrae body were evaluated, which indicated that the evacuation of drill chips is not essential for the capability to drill through the vertebrae body.
An analysis of the power loss due to friction at the needle tip of a 0.7mm Quincke needle has also been made, which indicated that there is little damage due to heat if the axial force is kept below 15N and the angular velocity below 40rad per second. By using a piezoresistive force sensor together with an encoder and current sensing, it is possible to continuously monitor the status of the needle in an MCU (Micro Controller Unit). From the MCU, the data is sent to Matlab and Simulink via USART (Universal Synchronous/Asynchronous Receiver/Transmitter) and USB (Universal Serial Bus), where it is used for analysis and control of functions.
All 25 0.7mm Quincke needles used in the test were able to drill through the vertebrae body of the spine of a domestic swine. The tests provide grounds for arguing that a Quincke needle can be used for drilling when performing lumbar puncture, without the risk of breaking the needle in the process.

The tests further shown that it is reasonable to conclude that it is possible to drill through vertebrae tissue, or calcified ligaments for that matter. The axial force never exceeded 1.04N until the cortical bone was struck in any of the tests, which is the force required to move the motor. This shows that the force required to insert the 0.7mm needle through the soft tissue is less than 1.04N. The measured force remained above 1.04N throughout the drilling process even through the spongy inner of the vertebrae body. As the needle exits on the other side of the vertebrae body, the force dropped below 1.04N, which implies that it is possible to use this as an indication to stop the drilling process. Due to the reason that the lumbar puncture process is not performed vertically in a real situation, but rather it is performed horizontally, the angle of insertion will affect the forces exerted on the sensor.
The results from the heat transfer calculations due to friction indicated that if the axial force is kept below 15N and if the angular velocity is less than 40rad per second, the generated heat for a 0.7mm Quincke needle would not cause significant damage to tissue in the vicinity of the drilling spot.

The present invention is not limited to the above-described preferred embodiments. Various alternatives, modifications and equivalents may be used. Therefore, the above embodiments should not be taken as limiting the scope of the invention, which is defined by the appending claims.

## Claims

1. A medical needle device (2) comprising
- a needle holder (4) structured to releasably hold a medical needle (6);
- a needle movement generator (8) configured to apply movement to the needle (6) via said needle holder (4), wherein said movement is one or many of a rotating or oscillating movement about a longitudinal axis A of the needle (6), and an oscillating movement along said longitudinal axis A, and
- a control unit (10) configured to determine and to control said movement of the needle (6) by applying a movement control signal (12), including control instructions, to the needle movement generator (8) to activate or deactivate said movement,
**characterized in that** the medical needle device (2) further comprises at least one parameter sensing member (14) configured to sense at least one parameter related to the needle (6), to generate at least one parameter signal (16) including parameter values representing at least one sensed parameter and to apply said parameter signal (16) to said control unit (10), wherein said control unit (10) is configured to receive said parameter signal (16) and to apply a set of control rules to said parameter values to determine said control instructions.

2. The medical needle device (2) according to claim 1, wherein said set of control rules includes at least one rule comprising to compare said parameter values to predetermined parameter thresholds, wherein said control unit (10) is configured to control needle movement in dependence of the result of said comparison.

3. The medical needle device (2) according to claim 1 or 2, wherein said parameter sensing member (14) is a force sensing member configured to sense a force to the needle (6) along said longitudinal axis A and in a proximal direction of the needle, and to generate a force sensor signal (18) including force values representing sensed forces and to apply said force sensor signal to said control unit (10), said control unit is configured to receive said force sensor signal (18) and to compare said force values to predetermined force thresholds, wherein said control unit is configured to control needle movement in dependence of the result of said comparison.

4. The medical needle device (2) according to claim 3, wherein if movement of the needle (6) is activated and a force value F is below a predetermined deactivation threshold (20) the control unit (10) is configured to generate said movement control signal (12) to the needle movement generator (8) to deactivate the movement.

5. The medical needle device (2) according to claim 3 or 4, wherein if movement of the needle (6) is deactivated and a force value F is above a predetermined activation threshold (22) the control unit (10) is configured to generate said movement control signal (12) to the needle movement generator (8) to activate the movement.

6. The medical needle device (2) according to any of claims 1-5, wherein said movement is a rotation about said longitudinal axis A.

7. The medical needle device (2) according to any of claims 1-5, wherein said movement is an oscillation about said longitudinal axis A, and wherein said oscillation is less than one revolution, and preferably less than half of a revolution.

8. The medical needle device (2) according to any of claims 1-7, wherein said movement is an oscillation along said longitudinal axis A having a predetermined stroke length.

9. The medical needle device (2) according to any of claims 1-8, wherein said parameter sensing member (14) is a torque sensing member configured to sense torque of the medical needle about said longitudinal axis, and to generate a torque sensor signal (24) including torque values T representing sensed torque and to apply said torque sensor signal (24) to said control unit (10), and wherein said control unit (10) is configured to control needle movement in dependence of sensed torque values.

10. The medical needle device (2) according to claim 9, wherein said control unit (10) is configured to receive said torque sensor signal (24) and to compare said torque values to predetermined torque threshold values, wherein if movement of the needle is activated and a torque value T is above a predetermined deactivation torque threshold (26) the control unit is configured to generate said movement control signal (12) to the needle movement generator (8) to deactivate the movement.

11. The medical needle device (2) according to claim 10, wherein said control unit (10) is configured to determine said movement control signal (12) in dependence of the present movement, preferably dependent of a present rotational speed of the needle, said force values or said torque values, such that temperature increase in tissue caused by needle movement is below a predefined temperature increase threshold.

12. The medical needle device (2) according to any of claims 1-11, comprising an activation member (28) to receive an activation command from an operator, and wherein the control unit (10) is configured to receive an activation signal (30) from said activation member (28) and to activate movement in dependence thereto.

13. The medical needle device (2) according to any of claims 1-12, further comprising a hand-held housing (32) structured to enclose said control unit (10) and said movement generator (8), the medical needle device further comprises a power source (34) configured to supply operating energy to the control unit and the movement generator.

14. The medical needle device (2) according to any of claims 1-13, configured to be applied during a spinal cord insertion procedure.

15. The medical needle device (2) according to any of claims 1-14, configured to be applied during a lumbar puncturing procedure.
